# EUROPÄISCHE PATENTSCHRIFT

(11) **EP 3 761 948 B1**
(45) Veröffentlichungstag und Bekanntmachung des Hinweises auf die Patenterteilung: **02.03.2022**
(21) Anmeldenummer: 19704778.0
(22) Anmeldetag: 08.02.2019
(51) Int. Cl.: A61K 8/41, A61Q 17/04, A61K 8/92

(54) **SONNENSCHUTZMITTEL MIT REDUZIERTER TEXTILVERFLECKUNG ENTHALTEND HYDRIERTES PFLANZENÖL UND DIETHYLAMINO HYDROXYBENZOYL HEXYL BENZOATE**
SUNSCREEN WITH A REDUCED TENDENCY TO STAIN TEXTILES, CONTAINING HYDROGENATED VEGETABLE OIL AND DIETHYLAMINO HYDROXYBENZOYL HEXYL BENZOATE
AGENT DE PROTECTION SOLAIRE AYANT UNE TENDANCE RÉDUITE À FORMER DES TACHES SUR LES TEXTILES ET CONTENANT DE L'HUILE VÉGÉTALE HYDROGÉNÉE ET DES DIÉTHYLAMINO HYDROXYBENZOYL HEXYL BENZOATES

(30) Priorität: 08.03.2018 DE 102018203496
(43) Veröffentlichungstag der Anmeldung: 13.01.2021
(73) Patentinhaber: Beiersdorf AG, 20253 Hamburg (DE)
(72) Erfinder: SPROCK, Sarah, 22297 Hamburg (DE); VOLBRICH, Heike, 22303 Hamburg (DE)
(74) Vertreter: Beiersdorf AG
(86) Internationale Anmeldenummer: PCT/EP2019/053131
(87) Internationale Veröffentlichungsnummer: WO 2019/170362

(56) Entgegenhaltungen:
- EP-A2- 1 529 521
- WO-A2-2007/128840
- DE-A1-102014 207 924
- US-A1- 2009 099 075
- US-A1- 2017 105 910
- DATABASE GNPD [Online] MINTEL; 16. August 2017 (2017-08-16), anonymous: "Sun Stick SPF 50", XP055577885, gefunden im www.gnpd.com Database accession no. 5025319
- DATABASE GNPD [Online] MINTEL; 29. September 2017 (2017-09-29), anonymous: "Day Serum M Silky Glow Makeup Base", XP55578436, gefunden im www.gnpd.com Database accession no. 5141673
- DATABASE GNPD [Online] MINTEL; 11. April 2017 (2017-04-11), anonymous: "Protect Day Cream SPF 50+ PA++++", XP055578441, gefunden im www.gnpd.com Database accession no. 4745707
- DATABASE GNPD [Online] MINTEL; 16. Februar 2017 (2017-02-16), anonymous: "Aquary Gel SPF 50+/PA++++", XP055578448, gefunden im www.gnpd.com Database accession no. 4625817
- DATABASE GNPD [Online] MINTEL; 27. Februar 2018 (2018-02-27), anonymous: "Lip Care SPF 15", XP055578461, gefunden im www.gnpd.com Database accession no. 5481037

## Beschreibung

Die vorliegende Erfindung betrifft eine kosmetische Zubereitung enthaltend hydriertes Rapsöl, und (2-[-4-(Diethylamino)-2-hydroxybenzoyl] Benzoesäurehexylester (INCI: Diethylamino Hydroxybenzoyl Hexyl Benzoate), sowie Verfahren und Verwendungen von hydriertem Pflanzenöl, insbesondere hydriertem Rapsöl, beim Schutz von Textilien vor dauerhaften Verfärbungen durch UV-Filter (insbesondere Diethylamino Hydroxybenzoyl Hexyl Benzoate) enthaltende kosmetische Zubereitungen.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter wie (2-[-4-(Diethylamino)-2-hydroxybenzoyl] Benzoesäurehexylester (INCI: Diethylamino Hydroxybenzoyl Hexyl Benzoate) hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch. Diesbezüglich lehrt DE102014207924, dass die Komplexbildner die Auswaschbarkeit von INCI: Diethylamino Hydroxybenzoyl Hexyl Benzoate erhöhen.

Es war daher die Aufgabe der vorliegenden Erfindung, die Nachteile des Standes der Technik zu beseitigen und eine kosmetische Zubereitung (insbesondere ein Sonnenschutzmittel) enthaltend nicht-wasserlösliche UVA-Filter wie (2-[-4-(Diethylamino)-2-hydroxybenzoyl] Benzoesäurehexylester (INCI: Diethylamino Hydroxybenzoyl Hexyl Benzoate) zu entwickeln, welche sich leichter aus den mit der Zubereitung kontaminierten Textilen herauswaschen lassen.

Überraschend gelöst wird diese Aufgabe durch eine kosmetische Zubereitung enthaltend (2-[-4-(Diethylamino)-2-hydroxybenzoyl] Benzoesäurehexylester (INCI: Diethylamino Hydroxybenzoyl Hexyl Benzoate) und hydriertes Rapsöl.

Überraschend gelöst wird diese Aufgabe ferner durch ein Verfahren zur Erhöhung der Auswaschbarkeit von organischen, öllöslichen UV-A Filtern und/oder organischen, öllöslichen Breitbandfiltern aus Textilien, die mit einer kosmetischen Zubereitung, welche (2-[-4-(Diethylamino)-2-hydroxybenzoyl] Benzoesäurehexylester (INCI: Diethylamino Hydroxybenzoyl Hexyl Benzoate) enthält, kontaminiert sind, dadurch gekennzeichnet, dass der kosmetischen Zubereitung hydriertes Rapsöl zugesetzt wird. Überraschend gelöst wird diese Aufgabe nicht zuletzt durch die Verwendung von hydriertem

Rapsöl in kosmetischen Zubereitungen enthaltend organische, öllösliche UV-A Filter und/oder Breitbandfilter zur Erhöhung der Auswaschbarkeit dieser UV-Filter aus mit der Zubereitung kontaminierten Textilien, wobei die kosmetische Zubereitung (2-[-4-(Diethylamino)-2-hydroxybenzoyl] Benzoesäurehexylester (INCI: Diethylamino Hydroxybenzoyl Hexyl Benzoate) enthält.

Im Rahmen der vorliegenden Offenbarung beziehen sich die Formulierungen "erfindungsgemäß", "erfindungsgemäße Zubereitung" etc. immer auf die erfindungsgemäßen Zubereitungen, Verfahren und Verwendungen, d.h. auch auf Zubereitungen, in denen die erfindungsgemäßen Verwendungen verwirklicht werden sowie Zubereitungen, mit denen das erfindungsgemäße Verfahren verwirklicht wird.

Vorteilhafte Ausführungsformen der vorliegenden Erfindung sind dadurch gekennzeichnet, dass die Zubereitung 4-(tert.-Butyl)-4'-methoxydibenzoylmethan und/oder 2,4-Bis-{[4-(2-ethyl-hexyl-oxy)-2-hydroxy]-phenyl}-6-(4-methoxyphenyl)-1,3,5-triazin (INCI Bis-Ethylhexyloxyphenol Methoxyphenyl Triazine) enthält.

Es ist erfindungsgemäß von Vorteil, wenn die Zubereitung hydriertes Rapsöl (INCI:
hydrogenated Rapeseed Oil) in einer Konzentration von 0,1 bis 10 Gewichts-%, bezogen auf das Gesamtgewicht der Zubereitung, enthält. Dabei ist ein Konzentrationsbereich von 0,1 bis 7 Gewichts-%, bezogen auf das Gesamtgewicht der Zubereitung, erfindungsgemäß bevorzugt. Ferner ist es erfindungsgemäß vorteilhaft, wenn die Zubereitung (2-[-4-(Diethylamino)-2-hydroxybenzoyl] Benzoesäurehexylester (INCI: Diethylamino Hydroxybenzoyl Hexyl Benzoate) in einer Konzentration von 0,1 bis 10 Gewichts-%, bezogen auf das Gesamtgewicht der Zubereitung enthält. Dabei ist ein Konzentrationsbereich von 0,1 bis 5 Gewichts-%, bezogen auf das Gesamtgewicht der Zubereitung, erfindungsgemäß bevorzugt.

Vorteilhaft im Sinne der vorliegenden Erfindung ist es, wenn das Gewichtsverhältnis von (2-[-4-(Diethylamino)-2-hydroxybenzoyl] Benzoesäurehexylester (INCI: Diethylamino Hydroxybenzoyl Hexyl Benzoate) zum hydrierten Rapsöl in der Zubereitung im Bereich von 0,01 : 1 bis 1:0,01 gewählt wird.

Es ist erfindungsgemäß, dass die Zubereitung frei ist von Propyl- und Butylparaben, 3-Iod-2-propinylbutylcarbamat, Hydroxyisohexyl 3-Cyclohexene Carboxalhdehyde (Lyral), 3-(4-Methylbenzyliden)-campher, 2-Hydroxy-4-methoxybenzophenon (Oxybenzon) und 4-Methoxyzimtsäure-2-ethylhexylester (Ethylhexyl Methoxycinnamate). Erfindungsgemäß bevorzugt wird darüber hinaus auch auf Methyl- und Ethylparaben sowie auf 2,6-Dihydroxy-4-methylbenzaldehyd (Atranol) und 3-Chlor-2,6-dihydroxy-4-methylbenzaldehyd (Chloratranol) verzichtet. Nicht zuletzt ist es erfindungsgemäß von Vorteil, wenn die Zubereitung frei ist von Octocrylen.

Erfindungsgemäß von Vorteil ist es, wenn die Zubereitung 4-(tert.-Butyl)-4'-methoxydiben-zoylmethan in einer Konzentration von 0,1 bis 5 Gewichts-%, bezogen auf das Gesamtgewicht der Zubereitung enthält.

Ferner ist es erfindungsgemäß vorteilhaft, wenn die Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) in einer Konzentration von 0,1 bis 10 Gewichts-%, bezogen auf das Gesamtgewicht der Zubereitung enthält. Dabei ist ein Konzentrationsbereich von 0,1 bis 7 Gewichts-%, bezogen auf das Gesamtgewicht der Zubereitung, erfindungsgemäß bevorzugt.

Vorteilhafte Ausführungsformen der vorliegenden Erfindung sind dadurch gekennzeichnet, dass die Zubereitung Ethanol in einer Konzentration von 0,1 bis 75 Gewichts-%, bezogen auf das Gesamtgewicht der Zubereitung enthält.

Die erfindungsgemäße Zubereitung kann in zwei unterschiedlichen Formen vorliegen: Als Emulsion und als ethanolische Lösung.

Liegt die Zubereitung als Emulsion vor, so ist es erfindungsgemäß von Vorteil, wenn diese Emulsion eine Öl-in-Wasser (O/W) Emulsion darstellt. In einem solchen Falle ist es erfindungsgemäß von Vorteil, wenn die Emulgatoren gewählt werden aus der Gruppe der Verbindungen Glycerylstearatcitrat, Glycerylstearat (selbstemulgierend), Stearinsäure, Stearatsalze, Polyglyceryl-3 methylglycose-distearat, Natriumcetearylsulfat, Kaliumcetylphosphat, Polyglyceryl-10 Stearate, Natriumstearoyl-glutamat.

Dabei sind die erfindungsgemäß vorteilhaften Ausführungsformen dadurch gekennzeichnet, dass der oder die Emulgatoren in einer Gesamtkonzentration von 0,2 bis 5 Gewichts-%, bezogen auf das Gesamtgewicht der Zubereitung eingesetzt werden.

Liegt die erfindungsgemäße Zubereitung hingegen als ethanolische Lösung vor, beträgt der Ethanolgehalt vorteilhaft von 20 bis 75 Gewichts-%, bezogen auf das Gesamtgewicht der Zubereitung.

Es ist erfindungsgemäß von Vorteil, wenn die Zubereitung Phenoxyethanol, Ethylhexylglycerin und/oder 4-Hydroxyacetophenon enthält. Dies gilt insbesondere für die erfindungsgemäßen Emulsionen.

Liegt die erfindungsgemäße Zubereitung in Form einer Emulsion vor, so kann die Wasserphase dieser Emulsion die hierfür üblichen Bestandteile enthalten. Darüber hinaus ist es erfindungsgemäß von Vorteil, wenn die Emulsion Propylenglykol, Butylenglycol, Glycerin, Elektrolyte, Selbstbräuner und/oder Verdickungsmittel enthält, beispielsweise Siliciumdioxide, Aluminiumsilikate oder Verbindungen aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Es ist erfindungsgemäß bevorzugt, wenn die erfindungsgemäße Zubereitung Silica Dimethyl Silylate enthält.

Für alle Zubereitungsformen der vorliegenden Erfindung ist es von Vorteil, wenn die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Vorteilhaft ist es auch, wenn die erfindungsgemäße Emulsion Stärkederivate wie Tapiocastärke, Distärkephosphat und/oder Aerosile^{®} enthält.

Ferner kann die erfindungsgemäße Zubereitung vorteilhaft Filmbildner enthalten. Vorteilhafte fettlösliche Filmbildner sind z. B., die Filmbildner aus der Gruppe der Polymere auf Basis von Polyvinylpyrrolidon (PVP)

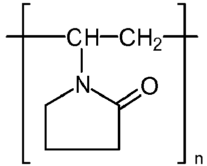

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Neben (2-[-4-(Diethylamino)-2-hydroxybenzoyl] Benzoesäurehexylester und gegebenenfalls 4-(tert.-Butyl)-4'-methoxydibenzoylmethan bzw. 2,4-Bis-{[4-(2-ethyl-hexyloxy)-2-hydroxy]-phenyl}-6-(4-methoxyphenyl)-1,3,5-triazin kann die erfindungsgemäße Zubereitung noch weitere für kosmetische Zubereitungen zugelassene UV-Filter enthalten. Dabei ist es erfindungsgemäß bevorzugt, wenn die Zubereitung einen oder mehrere UV-Filter aus der Gruppe der Verbindungen Homomenthylsalicylat, Ethylhexylsalicylat, Dioctylbutylamidotriazon (INCI: Diethylhexyl-Butamidotriazone), 2,4,6-Tris-[anilino-(p-carbo-2'-ethyl-1'-hexyloxy)]-1,3,5-triazin (INCI: Ethylhexyl Triazone), 2,2'-Methylen-bis-(6-(2H-benzotriazol-2-yl)-4-(1,1,3,3-tetramethylbutyl)-phenol), 2,4,6-Tribiphenyl-4-yl-1,3,5-triazin, Titandioxid, Phenylbenzimidazol-5-sulfonsäure und/oder deren Salze, [(3*Z*)-3-[[4-[(*Z*)-[7,7-Dimethyl-2-oxo-1-(sulfomethyl)-3-bicyclo[2.2.1]heptanyliden] methyl]phenyl]methyliden]-7,7-dimethyl-2-oxo-1-bicyclo[2.2.1]heptanyl]methan sulfonsäure (INCI: Terephthalylidene dicamphor sulfonic acid) und/oder dessen Salze enthält.

Es ist erfindungsgemäß von Vorteil, wenn die erfindungsgemäße Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, ß-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

Erfindungsgemäß vorteilhafte Ausführungsformen der vorliegenden Erfindung sind auch dadurch gekennzeichnet, dass die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

Es ist erfindungsgemäß von Vorteil, wenn die erfindungsgemäße Zubereitung einen oder mehrere der Parfümstoffe gewählt aus der Liste der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C, Butylphenylmethyl-propionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Ethylenbrassylat, Evernia Furfuracea Extract, Evernia Prunastri Extract, Farnesol, Guajakholzöl, Geraniol, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin, enthält.

Vorteilhafte Ausführungsformen der vorliegenden Erfindung sind dadurch gekennzeichnet, dass die Zubereitung Gumen und/oder Cellulose-Derivate enthält. Erfindungsgemäß bevorzugte Gumen sind Welan Gum, Sclerotium Gum und insbesondere Xanthan Gum.

Erfindungsgemäß bevorzugte Cellulose-Derivate sind mikrokristalline Cellulose und insbesondere Carboxymethylcellulose (Cellulose Gum).

Es ist vorteilhaft im Sinne der vorliegenden Erfindung, wenn die Zubereitung ein oder mehrere der Säure- bzw. Salzverbindungen aus der Gruppe 1-Hydroxyethan-(1,1-diphosphonsäure)/ HEDP, Aminotrimethylenphosphonsäure/ ATMP, Diethylentriaminpenta(methylenphosphonsäure)/ DTPMP, Ethylendiamintetra(methylenphosphonsäure/ EDTMP, Phosphonobutan-tricarbonsäure/ PBTC, Iminodisuccinat, Natriumpolyphosphat, Tetranatriumpyrophosphat, Hydroxamsäure, Polygalacturonsäure, Bernsteinsäure, Ameisensäure, Äpfelsäure, Ethylendiamintetraessigsäure (EDTA) und/oder deren Alkalisalze und/ oder deren Amin-N-Oxide enthält.

Erfindungsgemäß bevorzugt ist es dabei, wenn die Zubereitung die Natriumsalze der Ethylendiamintetraessigsäure und/oder der Iminodibernsteinsäure enthält. Besonders bevorzugt ist die Kombination aus den Natriumsalzen der Ethylendiamintetraessigsäure und der Iminodibernsteinsäure.

### Vergleichsversuch

Mit Hilfe des folgenden Vergleichsversuches konnte der erfinderische Effekt beispielhaft belegt werden: Es wurden die folgenden Rezepturen hergestellt und die Auswaschbarkeit aus Textilien bestimmt.

| **INCI** | **Referenz** | **+ Hydrogenated Rapeseed Oil** |
|---|---|---|
| Tocopheryl Acetate | 0,06 | 0,06 |
| Hydroxyacetophenone | 0,4 | 0,4 |
| Panthenol + Aqua | 1,4 | 1,4 |
| C12-15 Alkyl Benzoate | 2,5 | 2,0 |
| Butylene Glycol Dicaprylate/Dicaprate | 2,5 | 2,0 |
| Hydrogenated Rapeseed Oil | 0 | 1,0 |
| Sodium Stearoyl Glutamate | 0,25 | 0,25 |
| Silica Dimethyl Silylate | 1,0 | 1,0 |
| VP/Hexadecene Copolymer | 0,5 | 0,5 |
| Parfum | 0,4 | 0,4 |
| Glycerin + Aqua | 7,5 | 7,5 |
| Aqua + Sodium Hydroxide | 0,014 | 0,014 |
| Phenoxyethanol | 0,5 | 0,5 |
| Behenyl Alcohol | 0,9 | 0,9 |
| Stearyl Alcohol | 0,9 | 0,9 |
| Cellulose Gum | 0,5 | 0,5 |
| Xanthan Gum | 0,4 | 0,4 |
| Aqua | 48,776 | 48,776 |
| Alcohol Denat. + Aqua | 5,0 | 5,0 |
| Aqua + Trisodium EDTA | 1,0 | 1,0 |
| Tetrasodium Iminodisuccinate | 0,75 | 0,75 |
| Diethylamino Hydroxybenzoyl Hexyl Benzoate | 0,5 | 0,5 |
| Homosalate | 9,5 | 9,5 |
| Ethylhexyl Salicylate | 2,0 | 2,0 |
| Bis-Ethylhexyloxyphenol Methoxyphenyl Triazine | 3,5 | 3,5 |
| Ethylhexyl Triazone | 2,0 | 2,0 |
| Butyl Methoxydibenzoylmethane | 4,75 | 4,75 |
| Titanium Dioxide (nano) + Silica + Dimethicone | 2,0 | 2,0 |
| Phenylbenzimidazole Sulfonic Acid | 0,5 | 0,5 |

### Messung der Auswaschbarkeit

Es wurden die beiden Sonnenschutzemulsionen hinsichtlich der Bildung von gelben Flecke über einen in vitro Auftragungs-/ Waschzyklus untersucht. Es wurden dabei weiße vorgewaschene Baumwollmonitore (100% Baumwolle) verwendet. Dazu wurden je 3 mg/cm2 der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät DATACOLOR 800 (Datacolor International).

Messgeometrie: d/8°, SCE (Glanzkomponente ausgeschlossen)

Lichtart/Beobachter: D65/10°(entsprechend mittleres Tageslicht)

UV-Filterung: an D65 angepasst, Ganz/Griesser Methode

Messöffnung: LAV (30 mm Durchmesser)

Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen in einer Waschmaschine) (60°C, 2h, Ariel Compact Pulverwaschmittel,saubere Beiladung).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät DATACOLOR 800 (Datacolor International).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

### Ergebnis

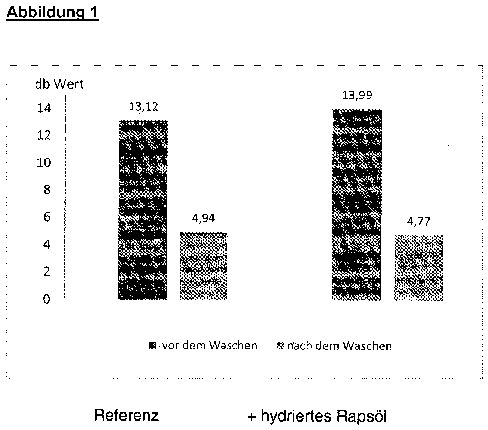

Während der db-Wert der Referenz-Rezeptur vor dem Waschen bei 13,12 liegt und nach dem Waschen auf 4,94 absinkt, geht dieser Wert bei der erfindungsgemäßen Zubereitung mit Hydrogenated Rapeseed Oil von 13,99 (vor dem Waschen) auf 4,77 (nach dem Waschen) zurück (siehe Abbildung 1).

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.
1) Sun Lotion SPF20

| **INCI** | **m(%)** |
|---|---|
| Tocopheryl Acetate | 0,1 |
| Hydroxyacetophenone | 0,4 |
| Panthenol | 1,5 |
| Ethylhexylglycerin | 0,3 |
| C12-15 Alkyl Benzoate | 5,0 |
| Isopropyl Palmitate | 6,0 |
| Butylene Glycol Dicaprylate/Dicaprate | 2,0 |
| Dimethicone | 0,9 |
| C18-38 Alkyl Hydroxystearoyl Stearate | 1,0 |
| Hydrogenated Rapeseed Oil | 0,75 |
| Hydrogenated Vegetable Oil | 0,3 |
| Polyglyceryl-10 Stearate | 0,4 |
| Silica Dimethyl Silylate | 1,0 |
| Triacontanyl PVP | 0,25 |
| VP/Hexadecene Copolymer | 0,25 |
| Parfum | 0,5 |
| Glycerin | 7,0 |
| Sodium Hydroxide | q.s |
| Behenyl Alcohol | 1,0 |
| Stearyl Alcohol | 1,0 |
| Acrylates/C10-30 Alkyl Acrylate Crosspolymer | 0,1 |
| Xanthan Gum | 0,4 |
| Aqua | add to 100 |
| Alcohol Denat. | 4,0 |
| Trisodium EDTA | 0,25 |
| Diethylamino Hydroxybenzoyl Hexyl Benzoate | 0,4 |
| Ethylhexyl Salicylate | 3,0 |
| Bis-Ethylhexyloxyphenol Methoxyphenyl Triazine | 0,33 |
| Ethylhexyl Triazone | 2,0 |
| Butyl Methoxydibenzoylmethane | 3,2 |
| Phenylbenzimidazole Sulfonic Acid | 0,8 |

2) Sun Lotion SPF 30

| **INCI** | **m(%)** |
|---|---|
| Tocopheryl Acetate | 0,06 |
| Hydroxyacetophenone | 0,4 |
| Panthenol | 1,1 |
| C12-15 Alkyl Benzoate | 5,0 |
| Isopropyl Palmitate | 6,0 |
| Butylene Glycol Dicaprylate/Dicaprate | 2,0 |
| Hydrogenated Rapeseed Oil | 1,0 |
| Sodium Stearoyl Glutamate | 0,25 |
| Silica Dimethyl Silylate | 1,0 |
| VP/Hexadecene Copolymer | 0,5 |
| Parfum | 0,4 |
| Glycerin | 7,5 |
| Sodium Hydroxide | q.s |
| Phenoxyethanol | 0,5 |
| Behenyl Alcohol | 1,0 |
| Stearyl Alcohol | 1,0 |
| Cellulose Gum | 0,35 |
| Xanthan Gum | 0,4 |
| Aqua | add to 100 |
| Alcohol Denat. | 5,0 |
| Trisodium EDTA | 0,2 |
| Tetrasodium Iminodisuccinate | 0,5 |
| Diethylamino Hydroxybenzoyl Hexyl Benzoate | 2,5 |
| Ethylhexyl Salicylate | 2,5 |
| Bis-Ethylhexyloxyphenol Methoxyphenyl Triazine | 2,2 |
| Ethylhexyl Triazone | 0,75 |
| Butyl Methoxydibenzoylmethane | 2,4 |
| Titanium Dioxide (nano) + Silica + Dimethicone | 3,0 |
| Phenylbenzimidazole Sulfonic Acid | 0,5 |

3) Sun Spray SPF 50+

| **INCI** | **m(%)** |
|---|---|
| Tocopheryl Acetate | 0,06 |
| Panthenol | 1,0 |
| Ethylhexylglycerin | 0,3 |
| Hydrogenated Rapeseed Oil | 0,5 |
| Dibutyl Adipate | 3,0 |
| Butylene Glycol Dicaprylate/Dicaprate | 0,5 |
| Copernicia Cerifera Cera | 1,5 |
| Sucrose Polystearate + Hydrogenated Polyisobutene | 1,0 |
| Polyg lyce ryl-4 Diisostearate/Polyhydroxystearate/Sebacate | 0,5 |
| Sodium Stearoyl Glutamate | 0,4 |
| VP/Hexadecene Copolymer | 1,0 |
| Parfum | 0,5 |
| Glycerin | 5,0 |
| Sodium Hydroxide | q.s |
| Phenoxyethanol | 0,5 |
| Cellulose Gum | 0,5 |
| Acrylates/C10-30 Alkyl Acrylate Crosspolymer | 0,1 |
| Xanthan Gum | 0,15 |
| Microcrystalline Cellulose + Cellulose Gum | 1,0 |
| Aqua | Add to 100 |
| Alcohol Denat. | 5,0 |
| Tetrasodium Iminodisuccinate | 0,75 |
| Trisodium EDTA | 0,2 |
| Homosalate | 8,0 |
| Diethylamino Hydroxybenzoyl Hexyl Benzoate | 1,0 |
| Ethylhexyl Salicylate | 8,5 |
| Bis-Ethylhexyloxyphenol Methoxyphenyl Triazine | 3,2 |
| Ethylhexyl Triazone | 2,8 |
| Butyl Methoxydibenzoylmethane | 5,00 |
| Phenylbenzimidazole Sulfonic Acid | 0,5 |

## Patentansprüche

1. Kosmetische Zubereitung enthaltend
(2-[-4-(Diethylamino)-2-hydroxybenzoyl] Benzoesäurehexylester (INCI: Diethylamino Hydroxybenzoyl Hexyl Benzoate) und
hydriertes Pflanzenöl (INCI: hydrogenated Vegetable Oil), wobei die Zubereitung als hydriertes Pflanzenöl (INCI hydrogenated Vegetable Oil) hydriertes Rapsöl (INCI: hydrogenated Rapeseed Oil) enthält und die Zubereitung frei ist von Propyl- und Butylparaben, 3-Iod-2-propinylbutylcarbamat, Hydroxyisohexyl 3-Cyclohexene Carboxalhdehyde (Lyral), 3-(4-Methylbenzyliden)-campher, 2-Hydroxy-4-methoxybenzophenon (Oxybenzon) und 4-Methoxyzimtsäure-2-ethylhexylester (Ethylhexyl Methoxycinnamate).

2. Verfahren zur Erhöhung der Auswaschbarkeit von organischen, öllöslichen UV-A Filtern und/oder organischen, öllöslichen Breitbandfiltern aus Textilien, die mit einer kosmetischen Zubereitung, welche (2-[-4-(Diethylamino)-2-hydroxybenzoyl] Benzoesäurehexylester (INCI: Diethylamino Hydroxybenzoyl Hexyl Benzoate) enthält, kontaminiert sind, **dadurch gekennzeichnet, dass** der kosmetischen Zubereitung hydriertes Pflanzenöl (INCI hydrogenated Vegetable Oil) zugesetzt wird, wobei die Zubereitung als hydriertes Pflanzenöl (INCI hydrogenated Vegetable Oil) hydriertes Rapsöl (INCI: hydrogenated Rapeseed Oil) enthält und die Zubereitung frei ist von Propyl- und Butylparaben, 3-Iod-2-propinylbutylcarbamat, Hydroxyisohexyl 3-Cyclohexene Carboxalhdehyde (Lyral), 3-(4-Methylbenzyliden)-campher, 2-Hydroxy-4-methoxybenzophenon (Oxybenzon) und 4-Methoxyzimtsäure-2-ethylhexylester (Ethylhexyl Methoxycinnamate).

3. Verwendung von hydriertem Pflanzenöl (INCI hydrogenated Vegetable Oil) in kosmetischen Zubereitungen enthaltend organische, öllösliche UV-A Filter und/oder Breitbandfilter zur Erhöhung der Auswaschbarkeit dieser UV-Filter aus mit der Zubereitung kontaminierten Textilien, wobei die kosmetische Zubereitung (2-[-4-(Diethylamino)-2-hydroxybenzoyl] Benzoesäurehexylester (INCI: Diethylamino Hydroxybenzoyl Hexyl Benzoate) enthält, wobei die Zubereitung als hydriertes Pflanzenöl (INCI hydrogenated Vegetable Oil) hydriertes Rapsöl (INCI: hydrogenated Rapeseed Oil) enthält.

4. Kosmetische Zubereitung, Verfahren oder Verwendung nach einem der vorhergehenden Ansprüche, **dadurch gekennzeichnet, dass** die Zubereitung 4-(tert.-Butyl)-4'-methoxydi-benzoylmethan und/oder 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine) enthält.

5. Kosmetische Zubereitung, Verfahren oder Verwendung nach einem der vorhergehenden Ansprüche, **dadurch gekennzeichnet, dass** die Zubereitung als hydriertes Pflanzenöl (INCI hydrogenated Vegetable Oil) ausschließlich hydriertes Rapsöl (INCI: hydrogenated Rapeseed Oil) enthält.

6. Kosmetische Zubereitung, Verfahren oder Verwendung nach einem der vorhergehenden Ansprüche, **dadurch gekennzeichnet, dass** die Zubereitung hydriertes Rapsöl (INCI: hydrogenated Rapeseed Oil) in einer Konzentration von 0,1 bis 10 Gewichts-%, bezogen auf das Gesamtgewicht der Zubereitung, enthält.

7. Kosmetische Zubereitung, Verfahren oder Verwendung nach einem der vorhergehenden Ansprüche, **dadurch gekennzeichnet, dass** die Zubereitung (2-[-4-(Diethylamino)-2-hydroxybenzoyl] Benzoesäurehexylester (INCI: Diethylamino Hydroxybenzoyl Hexyl Benzoate) in einer Konzentration von 0,1 bis 10 Gewichts-%, bezogen auf das Gesamtgewicht der Zubereitung enthält.

8. Kosmetische Zubereitung, Verfahren oder Verwendung nach einem der vorhergehenden Ansprüche, **dadurch gekennzeichnet, dass** das Gewichtsverhältnis von(2-[-4-(Diethylamino)-2-hydroxybenzoyl] Benzoesäurehexylester (INCI: Diethylamino Hydroxybenzoyl Hexyl Benzoate) zum hydrierten Rapsöl in der Zubereitung im Bereich von 0,01:1 bis 1:0,01 gewählt wird.

9. Kosmetische Zubereitung, Verfahren oder Verwendung nach einem der vorhergehenden Ansprüche, **dadurch gekennzeichnet, dass** die Zubereitung 4-(tert.-Butyl)-4'-methoxydibenzoylmethan in einer Konzentration von 0,1 bis 5 Gewichts-%, bezogen auf das Gesamtgewicht der Zubereitung enthält.

10. Kosmetische Zubereitung, Verfahren oder Verwendung nach einem der vorhergehenden Ansprüche, **dadurch gekennzeichnet, dass** die Zubereitung 2,4-Bis-{[4-(2-ethyl-hexyl-oxy)-2-hydroxy]-phenyl}-6-(4-methoxyphenyl)-1,3,5-triazin (INCI: Bis-Ethylhexyloxyphenol Methoxyphenyl Triazin) in einer Konzentration von 0,1 bis 10 Gewichts-%, bezogen auf das Gesamtgewicht der Zubereitung enthält.

11. Verwendung nach einem der vorhergehenden Ansprüche, **dadurch gekennzeichnet, dass** die Zubereitung frei ist von Propyl- und Butylparaben, 3-Iod-2-propinylbutylcarbamat, Hydroxyisohexyl 3-Cyclohexene Carboxalhdehyde (Lyral), 3-(4-Methylbenzyliden)-campher, 2-Hydroxy-4-methoxybenzophenon (Oxybenzon) und 4-Methoxyzimtsäure-2-ethylhexylester (Ethylhexyl Methoxycinnamate).

12. Kosmetische Zubereitung, Verfahren oder Verwendung nach einem der vorhergehenden Ansprüche, **dadurch gekennzeichnet, dass** die Zubereitung Ethanol in einer Konzentration von 0,1 bis 75 Gewichts-%, bezogen auf das Gesamtgewicht der Zubereitung enthält.

13. Kosmetische Zubereitung, Verfahren oder Verwendung nach einem der vorhergehenden Ansprüche, **dadurch gekennzeichnet, dass** die Zubereitung Phenoxyethanol, Ethylhexylglycerin und/oder 4-Hydroxyacetophenon enthält.

14. Kosmetische Zubereitung, Verfahren oder Verwendung nach einem der vorhergehenden Ansprüche, **dadurch gekennzeichnet, dass** die Zubereitung einen oder mehrere UV-Filter aus der Gruppe der Verbindungen Homomenthylsalicylat, Ethylhexylsalicylat, Dioctylbutylamidotriazon (INCI: Diethylhexyl-Butamidotriazone), 2,4,6-Tris-[anilino-(p-carbo-2'-ethyl-1'-hexyloxy)]-1,3,5-triazin (INCI: Ethylhexyl Triazone), 2,2'-Methylen-bis-(6-(2H-benzotriazol-2-yl)-4-(1,1,3,3-tetramethylbutyl)-phenol), 2,4,6-Tribiphenyl-4-yl-1,3,5-triazin, Titandioxid, Phenylbenzimidazol-5-sulfonsäure und/oder deren Salze, [(3Z)-3-[[4-[(Z)-[7,7-Dimethyl-2-oxo-1-(sulfomethyl)-3-bicyclo[2.2.1]heptanyliden] methyl]phenyl]methyliden]-7,7-dimethyl-2-oxo-1-bicyclo[2.2.1]heptanyl]methan sulfonsäure (INCI: Terephthalylidene dicamphor sulfonic acid) und/oder dessen Salze enthält.

15. Kosmetische Zubereitung, Verfahren oder Verwendung nach einem der vorhergehenden Ansprüche, **dadurch gekennzeichnet, dass** die Zubereitung Gumen und/oder Cellulose-Derivate enthält.

16. Kosmetische Zubereitung, Verfahren oder Verwendung nach einem der vorhergehenden Ansprüche, **dadurch gekennzeichnet, dass** die Zubereitung die Natriumsalze der Ethylendiamintetraessigsäure und/oder der Iminodibernsteinsäure enthält.

## Claims

1. Cosmetic preparation comprising
hexyl 2-[4-(diethylamino)-2-hydroxybenzoyl]benzoate (INCI: Diethylamino Hydroxybenzoyl Hexyl Benzoate) and
hydrogenated vegetable oil (INCI: Hydrogenated Vegetable Oil), wherein the preparation comprises hydrogenated rapeseed oil (INCI: Hydrogenated Rapeseed Oil) as hydrogenated vegetable oil (INCI: Hydrogenated Vegetable Oil) and the preparation is free from propyl paraben and butyl paraben, 3-iodo-2-propynyl butylcarbamate, hydroxyisohexylcyclohex-3-enecarboxaldehyde (Lyral), 3-(4-methylbenzylidene)camphor, 2-hydroxy-4-methoxybenzophenone (oxybenzone) and 2-ethylhexyl 4-methoxycinnamate (Ethylhexyl Methoxycinnamate).

2. Method for increasing the ability of organic, oil-soluble UVA filters and/or organic, oil-soluble broadband filters to be washed out of textiles which have been contaminated with a cosmetic preparation comprising hexyl 2-[4-(diethylamino)-2-hydroxybenzoyl]benzoate (INCI: Diethylamino Hydroxybenzoyl Hexyl Benzoate), **characterized in that** hydrogenated vegetable oil (INCI: Hydrogenated Vegetable Oil) is added to the cosmetic preparation, wherein the preparation comprises hydrogenated rapeseed oil (INCI: Hydrogenated Rapeseed Oil) as hydrogenated vegetable oil (INCI: Hydrogenated Vegetable Oil) and the preparation is free from propyl paraben and butyl paraben, 3-iodo-2-propynyl butylcarbamate, hydroxyisohexylcyclohex-3-enecarboxaldehyde (Lyral), 3-(4-methylbenzylidene)camphor, 2-hydroxy-4-methoxybenzophenone (oxybenzone) and 2-ethylhexyl 4-methoxycinnamate (Ethylhexyl Methoxycinnamate).

3. Use of hydrogenated vegetable oil (INCI: Hydrogenated Vegetable Oil) in cosmetic preparations comprising organic, oil-soluble UVA filters and/or broadband filters for increasing the ability of said UV filters to be washed out of textiles contaminated with the preparation, wherein the cosmetic preparation comprises hexyl 2-[4-(diethylamino)-2-hydroxybenzoyl]benzoate (INCI: Diethylamino Hydroxybenzoyl Hexyl Benzoate), wherein the preparation comprises hydrogenated rapeseed oil (INCI: Hydrogenated Rapeseed Oil) as hydrogenated vegetable oil (INCI: Hydrogenated Vegetable Oil).

4. Cosmetic preparation, method or use according to any of the preceding claims, **characterized in that** the preparation comprises 4-(tert-butyl)-4'-methoxydibenzoylmethane and/or 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine).

5. Cosmetic preparation, method or use according to any of the preceding claims, **characterized in that** the preparation comprises exclusively hydrogenated rapeseed oil (INCI: Hydrogenated Rapeseed Oil) as hydrogenated vegetable oil (INCI: Hydrogenated Vegetable Oil).

6. Cosmetic preparation, method or use according to any of the preceding claims, **characterized in that** the preparation comprises hydrogenated rapeseed oil (INCI: Hydrogenated Rapeseed Oil) at a concentration of 0.1 to 10% by weight, based on the total weight of the preparation.

7. Cosmetic preparation, method or use according to any of the preceding claims, **characterized in that** the preparation comprises hexyl 2-[4-(diethylamino)-2-hydroxybenzoyl]benzoate (INCI: Diethylamino Hydroxybenzoyl Hexyl Benzoate) at a concentration of 0.1 to 10% by weight, based on the total weight of the preparation.

8. Cosmetic preparation, method or use according to any of the preceding claims, **characterized in that** the ratio by weight of hexyl 2-[4-(diethylamino)-2-hydroxybenzoyl]benzoate (INCI: Diethylamino Hydroxybenzoyl Hexyl Benzoate) to the hydrogenated rapeseed oil in the preparation is selected in the range from 0.01:1 to 1:0.01.

9. Cosmetic preparation, method or use according to any of the preceding claims, **characterized in that** the preparation comprises 4-(tert-butyl)-4'-methoxydibenzoylmethane at a concentration of 0.1 to 5% by weight, based on the total weight of the preparation.

10. Cosmetic preparation, method or use according to any of the preceding claims, **characterized in that** the preparation comprises 2,4-bis-{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) at a concentration of 0.1 to 10% by weight, based on the total weight of the preparation.

11. Use according to any of the preceding claims, **characterized in that** the preparation is free from propyl paraben and butyl paraben, 3-iodo-2-propynyl butylcarbamate, hydroxyisohexylcyclohex-3-enecarboxaldehyde (Lyral), 3-(4-methylbenzylidene)camphor, 2-hydroxy-4-methoxybenzophenone (oxybenzone) and 2-ethylhexyl 4-methoxycinnamate (Ethylhexyl Methoxycinnamate).

12. Cosmetic preparation, method or use according to any of the preceding claims, **characterized in that** the preparation comprises ethanol at a concentration of 0.1 to 75% by weight, based on the total weight of the preparation.

13. Cosmetic preparation, method or use according to any of the preceding claims, **characterized in that** the preparation comprises phenoxyethanol, ethylhexylglycerin and/or 4-hydroxyacetophenone.

14. Cosmetic preparation, method or use according to any of the preceding claims, **characterized in that** the preparation comprises one or more UV filters from the group of compounds comprising homomenthyl salicylate, ethylhexyl salicylate, dioctylbutylamidotriazone (INCI: Diethylhexyl Butamidotriazone), 2,4,6-tris[anilino-(p-carbo-2'-ethyl-1'-hexyloxy)]-1,3,5-triazine (INCI: Ethylhexyl Triazone), 2,2'-methylenebis(6-(2H-benzotriazol-2-yl)-4-(1,1,3,3-tetramethylbutyl)phenol), 2,4,6-tribiphenyl-4-yl-1,3,5-triazine, titanium dioxide, phenylbenzimidazole-5-sulfonic acid and/or salts thereof, [(3Z)-3-[[4-[(Z)-[7,7-dimethyl-2-oxo-1-(sulfomethyl)-3-bicyclo[2.2.1]heptanylidene]methyl]phenyl]methyl-idene]-7,7-dimethyl-2-oxo-1-bicyclo[2.2.1]heptanyl]methanesulfonic acid (INCI: Terephthalylidene Dicamphor Sulfonic Acid) and/or salts thereof.

15. Cosmetic preparation, method or use according to any of the preceding claims, **characterized in that** the preparation comprises gums and/or cellulose derivatives.

16. Cosmetic preparation, method or use according to any of the preceding claims, **characterized in that** the preparation comprises the sodium salts of ethylenediaminetetracetic acid and/or of iminodisuccinic acid.

## Revendications

1. Préparation cosmétique contenant de l'ester d'hexyle d'acide (2-[-4-(diéthylamino)-2-hydroxybenzoyl]benzoïque (INCI: Diethylamino Hydroxybenzoyl Hexyl Benzoate) et une huile végétale hydrogénée (INCI : hydrogenated Vegetable Oil), la préparation contenant de l'huile de colza hydrogénée (INCI: hydrogenated Rapeseed Oil) en tant qu'huile végétale hydrogénée (INCI : hydrogenated Vegetable Oil) et la préparation étant exempte de propylparabène et de butylparabène, de 3-iodo-2-propinylbutylcarbamate, d'hydroxyisohexyl-3-cyclohexène-carboxalhdéhyde (lyral), de 3-(4-méthylbenzylidène)-camphre, de 2-hydroxy-4-méthoxybenzophénone (oxybenzone) et d'ester de 2-éthylhexyle d'acide 4-méthoxycinnamique (éthylhexyle méthoxycinnamate).

2. Procédé pour l'augmentation de la lessivabilité de filtres d'UV-A organiques, liposolubles et/ou de filtres à bande large organiques, liposolubles de textiles qui sont contaminés par une préparation cosmétique qui contient de l'ester d'hexyle d'acide (2-[-4-(diéthylamino)-2-hydroxybenzoyl]benzoïque (INCI: Diethylamino Hydroxybenzoyl Hexyl Benzoate), **caractérisé en ce qu'**une huile végétale hydrogénée (INCI : hydrogenated Vegetable Oil) est ajoutée à la préparation cosmétique, la préparation contenant de l'huile de colza hydrogénée (INCI: hydrogenated Rapeseed Oil) en tant qu'huile végétale hydrogénée (INCI : hydrogenated Vegetable Oil) et la préparation étant exempte de propylparabène et de butylparabène, de 3-iodo-2-propinylbutylcarbamate, d'hydroxyisohexyl-3-cyclohexène-carboxalhdéhyde (lyral), de 3-(4-méthylbenzylidène)-camphre, de 2-hydroxy-4-méthoxybenzophénone (oxybenzone) et d'ester de 2-éthylhexyle d'acide 4-méthoxycinnamique (éthylhexyle méthoxycinnamate).

3. Utilisation d'huile végétale hydrogénée (INCI : hydrogenated Vegetable Oil) dans des préparations cosmétiques contenant des filtres d'UV-A organiques, liposolubles et/ou des filtres à bande large organiques, liposolubles pour l'augmentation de la lessivabilité de ces filtres UV de textiles contaminés par la préparation, la préparation cosmétique contenant de l'ester d'hexyle d'acide (2-[-4-(diéthylamino)-2-hydroxybenzoyl]benzoïque (INCI : Diethylamino Hydroxybenzoyl Hexyl Benzoate), la préparation contenant de l'huile de colza hydrogénée (INCI: hydrogenated Rapeseed Oil) en tant qu'huile végétale hydrogénée (INCI : hydrogenated Vegetable Oil).

4. Préparation cosmétique, procédé ou utilisation selon l'une quelconque des revendications précédentes, caractérisé(e) en ce que la préparation contient du 4-(tert-butyl)-4'-méthoxydibenzoylméthane et/ou de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine).

5. Préparation cosmétique, procédé ou utilisation selon l'une quelconque des revendications précédentes, caractérisé(e) en ce que la préparation contient exclusivement de l'huile de colza hydrogénée (INCI: hydrogenated Rapeseed Oil) en tant qu'huile végétale hydrogénée (INCI : hydrogenated Vegetable Oil).

6. Préparation cosmétique, procédé ou utilisation selon l'une quelconque des revendications précédentes, caractérisé(e) en ce que la préparation contient de l'huile de colza hydrogénée (INCI: hydrogenated Rapeseed Oil) en une concentration de 0,1 à 10 % en poids, par rapport au poids total de la préparation.

7. Préparation cosmétique, procédé ou utilisation selon l'une quelconque des revendications précédentes, caractérisé(e) en ce que la préparation contient de l'ester d'hexyle d'acide (2-[-4-(diéthylamino)-2-hydroxybenzoyl]benzoïque (INCI : Diethylamino Hydroxybenzoyl Hexyl Benzoate) en une concentration de 0,1 à 10 % en poids, par rapport au poids total de la préparation.

8. Préparation cosmétique, procédé ou utilisation selon l'une quelconque des revendications précédentes, caractérisé(e) en ce que le rapport en poids de l'ester d'hexyle d'acide (2-[-4-(diéthylamino)-2-hydroxybenzoyl]benzoïque (INCI : Diethylamino Hydroxybenzoyl Hexyl Benzoate) sur l'huile de colza hydrogénée dans la préparation est choisi dans la plage de 0,01 : 1 à 1 : 0,01.

9. Préparation cosmétique, procédé ou utilisation selon l'une quelconque des revendications précédentes, caractérisé(e) en ce que la préparation contient du 4-(tert-butyl)-4'-méthoxydibenzoylméthane en une concentration de 0,1 à 5 % en poids, par rapport au poids total de la préparation.

10. Préparation cosmétique, procédé ou utilisation selon l'une quelconque des revendications précédentes, caractérisé(e) en ce que la préparation contient de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine) en une concentration de 0,1 à 10 % en poids, par rapport au poids total de la préparation.

11. Utilisation selon l'une quelconque des revendications précédentes, **caractérisée en ce que** la préparation est exempte de propylparabène et de butylparabène, de 3-iodo-2-propinylbutylcarbamate, d'hydroxyisohexyl-3-cyclohexène-carboxalhdéhyde (lyral), de 3-(4-méthylbenzylidène)-camphre, de 2-hydroxy-4-méthoxybenzophénone (oxybenzone) et d'ester de 2-éthylhexyle d'acide 4-méthoxycinnamique (éthylhexyle méthoxycinnamate).

12. Préparation cosmétique, procédé ou utilisation selon l'une quelconque des revendications précédentes, caractérisé(e) en ce que la préparation contient de l'éthanol en une concentration de 0,1 à 75 % en poids, par rapport au poids total de la préparation.

13. Préparation cosmétique, procédé ou utilisation selon l'une quelconque des revendications précédentes, caractérisé(e) la préparation contient du phénoxyéthanol, de l'éthylhexylglycérine et/ou de la 4-hydroxyacétophénone.

14. Préparation cosmétique, procédé ou utilisation selon l'une quelconque des revendications précédentes, caractérisé(e) la préparation contient un ou plusieurs filtres UV du groupe de composés comprenant le salicylate d'homomenthyle, le salicylate d'éthylhexyle, la dioctylbutylamidotriazone (INCI : Diethylhexyl-Butamidotriazone), la 2,4,6-tris-[anilino-(p-carbo-2'-éthyl-1'-hexyloxy)]-1,3,5-triazine (INCI : Ethylhexyl Triazone), le 2,2'-méthylène-bis-(6-(2H-benzotriazol-2-yl)-4-(1,1,3,3-tétraméthylbutyl)-phénol), la 2,4,6-tribiphényl-4-yl-1,3,5-triazine, le dioxyde de titane, l'acide phénylbenzimidazol-5-sulfonique et/ou ses sels, l'acide [(3Z)-3-[[4-[(Z)-[7,7-diméthyl-2-oxo-1-(sulfométhyl)-3-bicyclo[2.2.1]heptanylidène]méthyl]phényl]méthyli-dène]-7,7-diméthyl-2-oxo-1-bicyclo[2.2.1]heptanyl]méthanesulfonique (INCI : Terephthalylidene dicamphor sulfonic acid) et/ou ses sels.

15. Préparation cosmétique, procédé ou utilisation selon l'une quelconque des revendications précédentes, caractérisé(e) en ce que la préparation contient des gommes et/ou des dérivés de cellulose.

16. Préparation cosmétique, procédé ou utilisation selon l'une quelconque des revendications précédentes, caractérisé(e) la préparation contient les sels de sodium de l'acide éthylènediaminetétraacétique et/ou de l'acide iminodisuccinique.
